# EUROPEAN PATENT SPECIFICATION

(11) **EP 2 552 520 B1**
(45) Date of publication and mention of the grant of the patent: **25.11.2015**
(21) Application number: 11729702.8
(22) Date of filing: 24.03.2011
(51) Int. Cl.: A61M 5/32

(54) **PROTECTION DEVICE WITH INCREASED FUNCTIONALITY, PARTICULARLY FOR SYRINGES AND THE LIKE**
SCHUTZVORRICHTUNG MIT VERBESSERTER FUNKTIONALITÄT, INSBESONDERE FÜR SPRITZEN UND DERGLEICHEN
DISPOSITIF DE PROTECTION À FONCTIONNALITÉ ACCRUE, EN PARTICULIER POUR LES SERINGUES ET ANALOGUES

(30) Priority: 26.03.2010 IT MI20100506
(43) Date of publication of application: 06.02.2013
(73) Proprietor: MARGHERITA INVENTIONS S.R.L., 41038 San Felice sul Panaro (MO) (IT)
(72) Inventor: GIUBERCHIO, Carlo, 27100 Pavia (IT)
(74) Representative: Modiano, Micaela Nadia
(86) International application number: PCT/IB2011/051261
(87) International publication number: WO 2011/117840

(56) References cited:
- EP-A1- 0 361 139
- EP-A2- 0 192 453
- US-A- 4 781 697

## Description

### Technical Field

The present invention relates to a protection device with increased functionality, particularly for syringes and the like.

### Background Art

In the field of medical or laboratory instruments, needle instruments are known, such as syringes and the like, which are adapted to extract and/or inject liquids by means of a needle.

Since it can happen that the liquids handled with such needle instruments contain substances that are contaminant or harmful to humans, conventional needle instruments are generally provided with a covering cap that fits over the needle of the instrument, to protect it, and which can be removably fixed to the instrument in such a way as to avoid accidental injury to the operator or to avoid the operator coming into contact with the contaminant or harmful substances when the needle is not being used for performing extraction or injection operations.

More precisely, the covering cap has a cannula shape of length substantially equal to that of the needle to be covered and with at least one end perforated so as to allow the needle of the instrument to be inserted.

In this way, the needle of the instrument, which is integral with the instrument proper, can be repeatedly inserted into and extracted from the covering cap between one extraction and the next or between one injection and the next.

Typically, the recapping operation is performed manually by the operator, holding the covering cap in one hand and the needle instrument in the other hand.

During such operation, it can happen that inadvertently, the operator misses the entry hole of the covering cap, thus sending the needle into contact with the fingers of his/her hand, leading to the contamination thereof with the liquid being handled, or simply injuring himself/herself with the point of the needle.

To overcome this drawback, protection devices are known which consist of a tubular grip which is adapted to accommodate the protective cap of the needle instruments and which is provided, at one end thereof, with a protection pan perforated at the inner cavity of the grip in order to permit the insertion into and the extraction from the covering cap between one extraction and the next or between one injection and the next.

In this way, the operator's hand that holds the covering cap, by gripping the conventional protection device by its grip, is protected by the pan so as to prevent accidental injury or contamination.

Once the needle has been extracted from the respective covering cap, the cap remains accommodated in the grip of the protection device until the next recapping.

Conventional protection devices are not devoid of drawbacks, including the fact that they cannot be placed on a resting surface, for example a worktable, in a stable position if the needle instrument is inserted with its needle in the tubular grip.

Indeed, by placing it on an edge, there is the risk that the needle instrument will come out of the grip or that it will fall off the worktable it is resting on.

Devices suitable to offer protection to an operator from injury by contaminated syringe or hypodermic needles are known, for example, from the documents EP 0 361 139 A1, US 4 781 697 and EP 0 192 453 A2.

### Disclosure of the invention

The aim of the present invention is to provide a protection device with improved functionality, particularly for syringes and the like, which enables both its stable positioning on a resting surface even with the needle applied to the needle instrument and also the recapping of the needle even with one hand only.

Within this aim, an object of the present invention consists in providing a protection device with improved functionality that is simple to implement, easy to use, and at low cost.

This aim, as well as these and other objects which will become better apparent hereinafter, are achieved by a protection device, according to the present invention, that has the features set forth in claim 1.

### Brief description of the drawings

Further characteristics and advantages of the present invention will become better apparent from the description of a preferred, but not exclusive, embodiment of a protection device with improved functionality, particularly for syringes or the like, according to the invention, illustrated by way of nonlimiting example in the accompanying drawings, wherein:
Figure 1 is a perspective view of a preferred, but not exclusive, embodiment of a protection device with improved functionality, particularly for syringes and the like, according to the invention, showing a syringe during the insertion thereof into the protection device;
Figure 2 is a perspective view of the protection device and of the syringe shown in Figure 1 after insertion;
Figure 3 is a perspective view of the protection device and of the syringe shown in Figure 1 after insertion and with the protection device placed on a resting surface in a first stable position thereof;
Figure 4 is a perspective view of the protection device and of the syringe shown in Figure 1 during the extraction of the needle of the syringe from the protection cap;
Figure 5 is a perspective view of the protection device and of the cap shown in Figure 4 after extraction and with the protection device placed on a resting surface in a second stable position thereof;
Figure 6 is a perspective view of the protection device and of the cap of the syringe shown in Figure 1 after extraction of the syringe from the protection cap and with the protection device placed on a resting surface in the first stable position thereof;
Figure 7 is a perspective view of the protection device and of the syringe shown in Figure 1 during insertion of the needle of the syringe into the protection cap by means of one hand only with the protection device placed on a resting surface in the first stable position thereof;
Figure 8 is a perspective view of the protection device and of the syringe shown in Figure 1 during the recapping of the needle of the syringe in the protection cap;
Figure 9 is a perspective view of the protection device and of the syringe shown in Figure 1 during the removal of the syringe from the protection device;
Figure 10 is a perspective view of the protection device and of the syringe shown in Figure 1 during the decoupling of the needle accommodated in the protection cap from the syringe;
Figure 11 is a perspective view of the protection device and of the protection cap, accommodating the needle of the syringe, shown in Figure 1 during the removal of the protection cap from the protection device.

### Ways of carrying out the invention

With reference to the figures, the protection device with improved functionality, particularly for syringes and the like, generally designated with the reference numeral 1, comprises a supporting body 2 which is substantially disk-like.

In more detail, the supporting body 2 is also provided with a hole 3, arranged coaxially to the supporting body 2, for the insertion of the covering cap 4 of a needle 5 of a syringe 6 or the like.

As will be better described hereinafter, the hole 3 has a diameter that is substantially larger than the diameter of the central portion 4a of the covering cap 4 and substantially smaller than the diameter of the end portion 4b of the covering cap 4 at the end for the insertion of the needle 5 so as to ensure the engagement between the two.

According to the invention, the supporting body 2 has at least one flat face 7, arranged on the opposite side with respect to the face for the insertion of the covering cap 4 in the hole 3, for the resting contact of the supporting body 2 on a resting surface 8 or the like in a first stable position.

Advantageously, on the supporting body 2, on the opposite side with respect to the flat face 7, there is a face 9 provided with a raised perimetric containment rim 10.

In the center of the face 9, at the hole 3 and coaxially thereto, a collar 11 is provided, that extends from the face 9 and which acts as a guide element for vertically supporting the covering cap 4 with the supporting body 2 placed on the resting surface 8 with its flat face 7.

In more detail, the collar 11 extends substantially at right angles from the face 9 for a length that is shorter than the height of the outer perimetric rim 10, in order to permit the resting contact of the supporting body 2 on the side of the rim 10 on the resting surface 8 or the like, with the flat face 7 directed upward.

With such geometry, the collar 11 defines an abutment surface 12 upon which the flange 13 of the protection cap 4, if present, engages by contact.

If the flange 13 is not provided, the protection cap 4 having only a conicalness concordant with the direction of insertion in the hole 3, then the end portion 4b, being radially larger than the central portion 4a, engages by mechanical interference the hole 3.

The collar 11 has a length that is such as to allow the protection cap 4 to protrude for a predominant portion thereof from the face 7 of the device, when the device is lifted off the resting surface.

Operation of the protection device 1 with improved functionality, particularly for syringes and the like, is as follows.

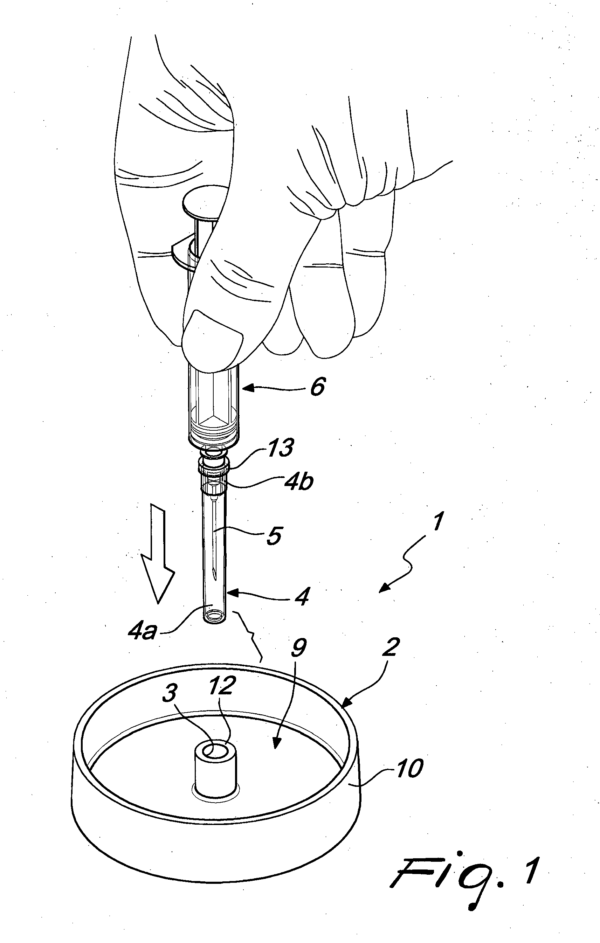

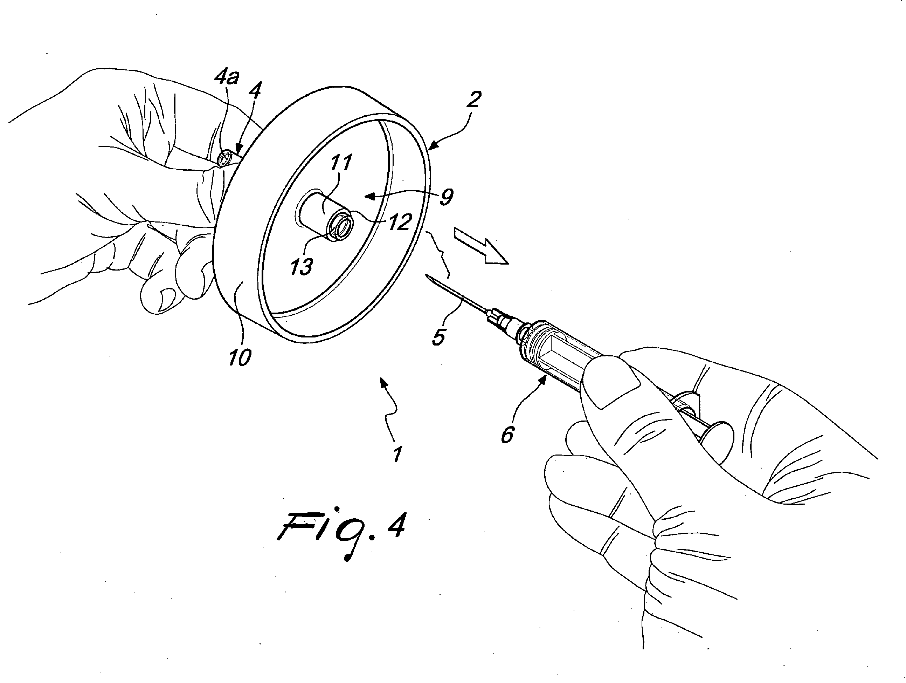

With particular reference to Figures 1 to 6, with the covering cap 4, which is fitted over so as to protect the needle 5 of the syringe 6, inserted in the hole 3 of the supporting body 2, it is possible to disengage the covering cap 4 from the syringe 6 by gripping it at its central portion 4a with the same hand with which the protection device 1 is supported and pulling, with the other hand, the syringe 6.

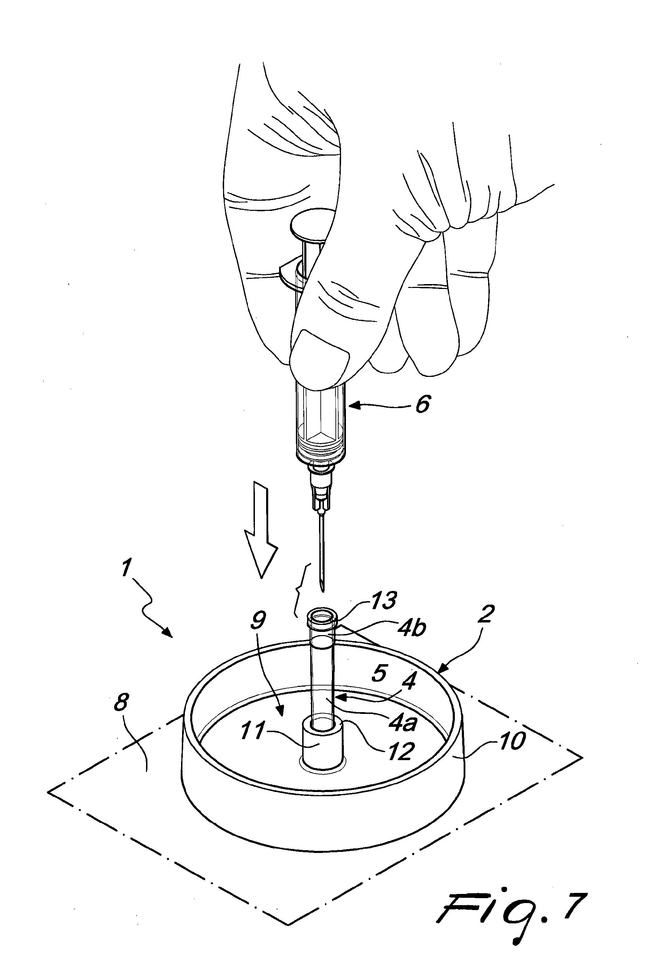

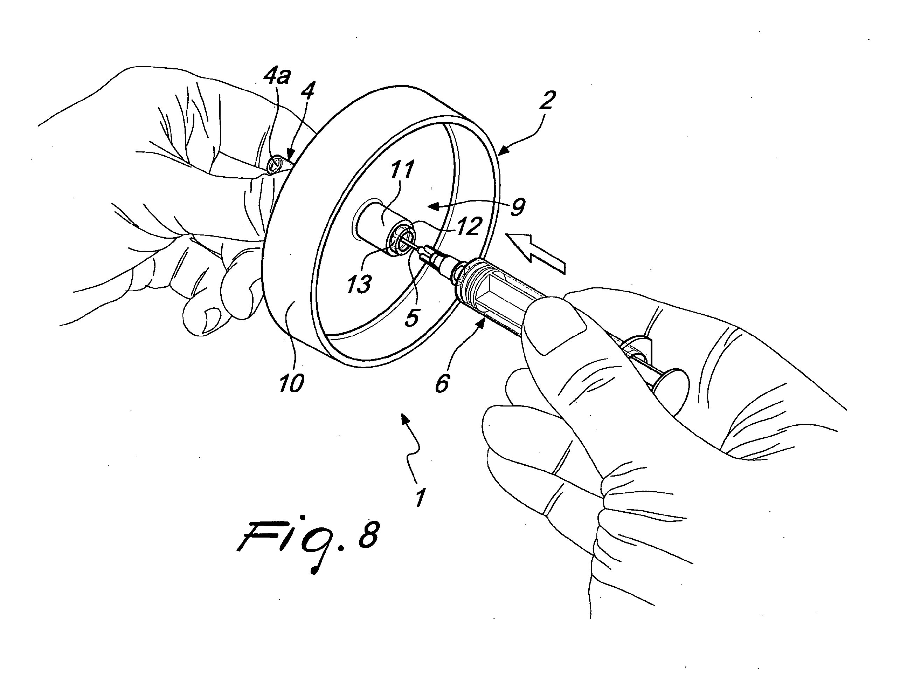

Similarly, as shown in Figures 7 and 8, once the withdrawal and injection operations are performed, by repeating the procedure just described in reverse it is possible to reinsert the needle 5 into the protection cap 4.

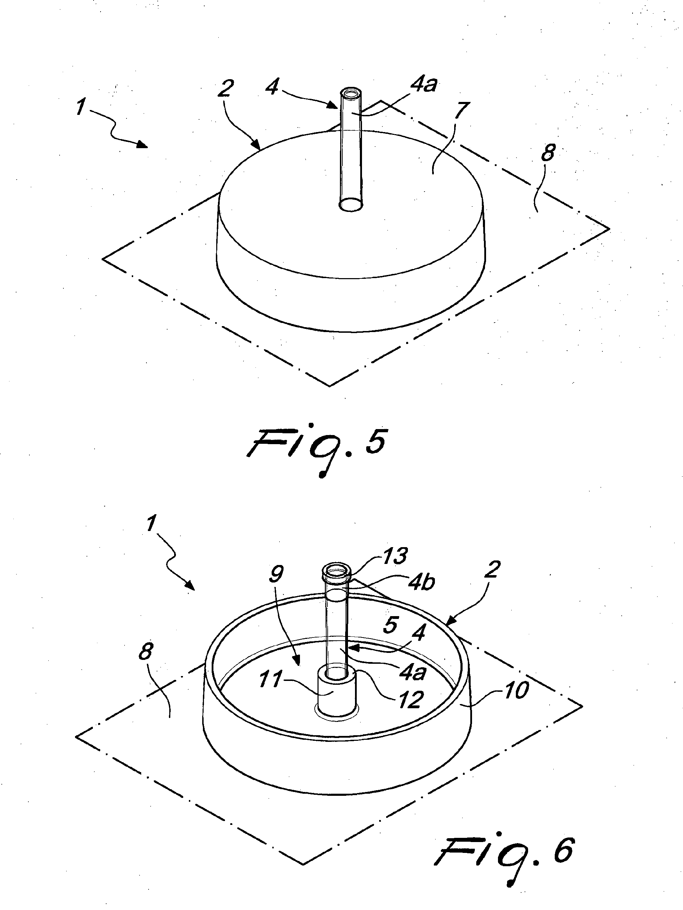

Advantageously, as shown in Figures 5 and 6, once the protection cap 4 is removed from the syringe 6, due to the shape of the protection device 1 it is possible to position it in two stable positions: a first in which the face 9 is turned downward, i.e. with the flat face 7 resting on the resting surface 8, and a second with the face 9 turned upward, i.e. with the outer rim 10 resting on the resting surface 8.

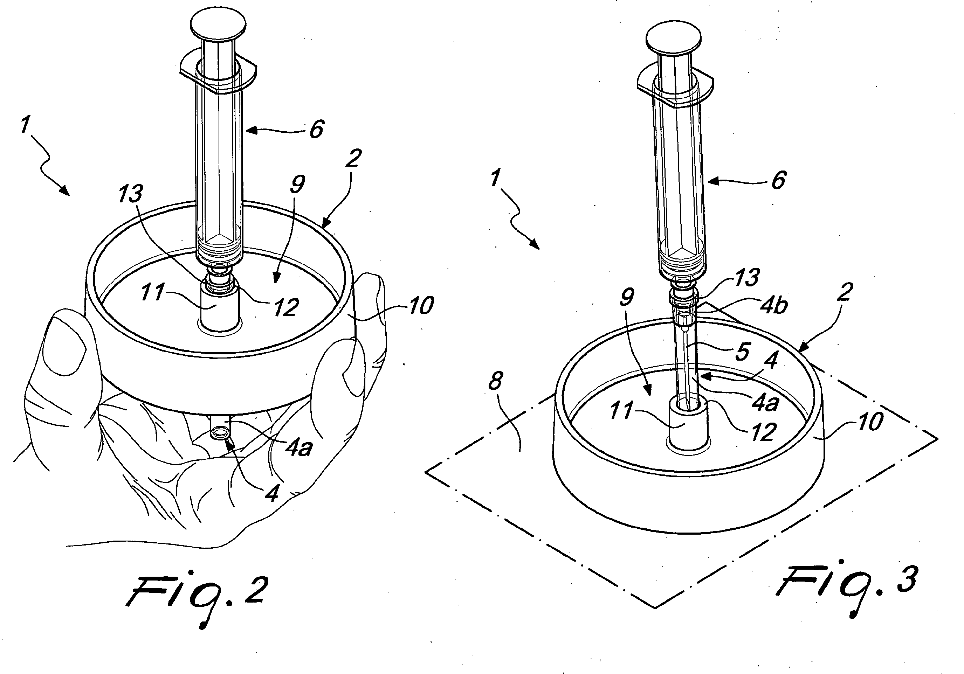

In more detail, as shown in Figure 7, with the protection device 1 arranged in the first stable position it is possible to perform the operation of recapping the needle 5 of the syringe 6 with one hand only, thus ending up with the syringe 6 and with the needle 5 reinserted in the protection cap 4 which in turn is inserted in the hole 3; in this way, the syringe 6 is held in a vertical position thanks to the support provided by the collar 11 as shown in Figure 3.

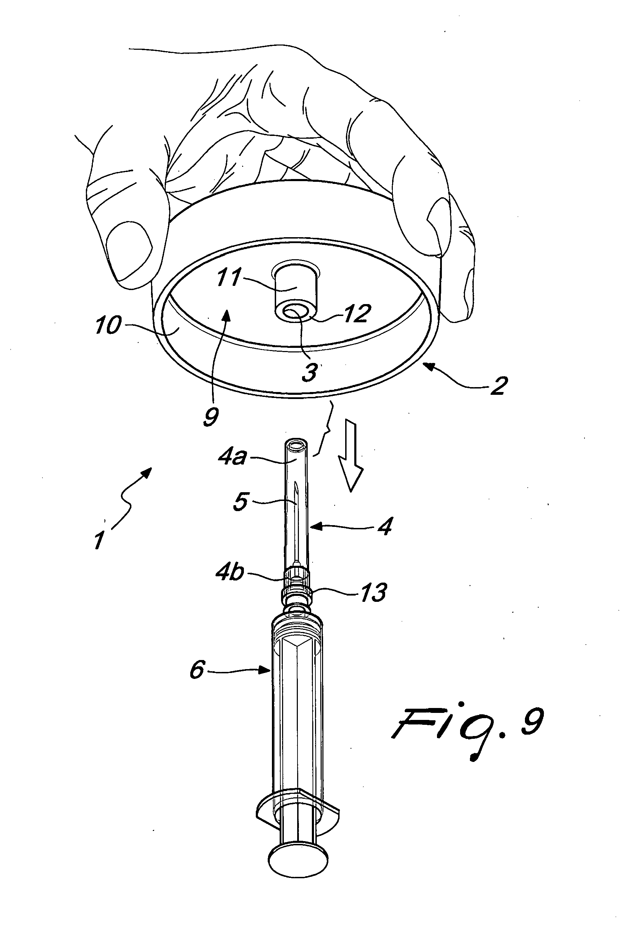

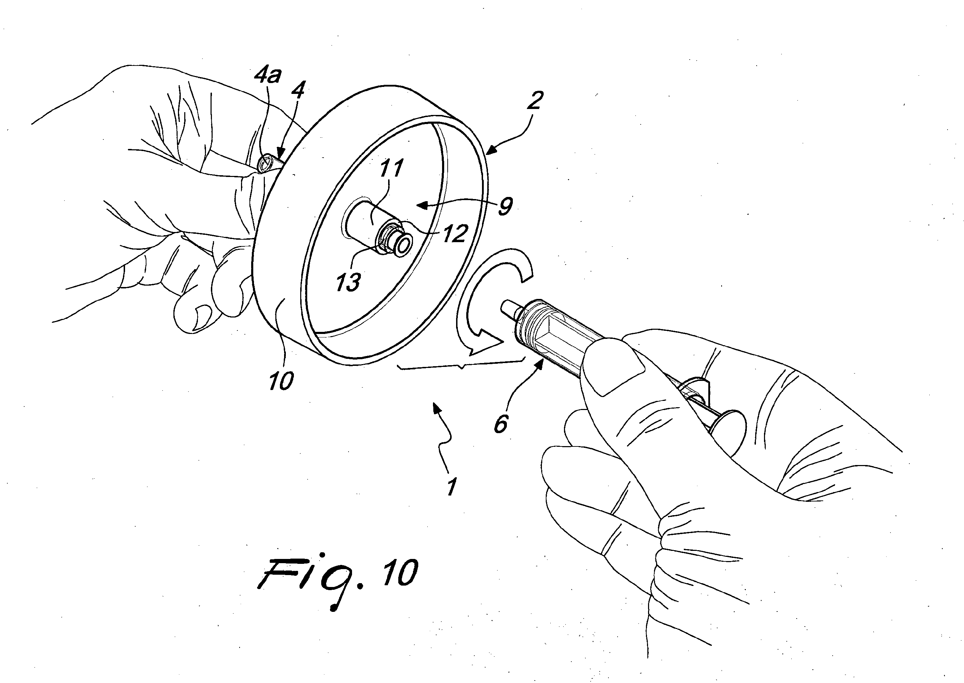

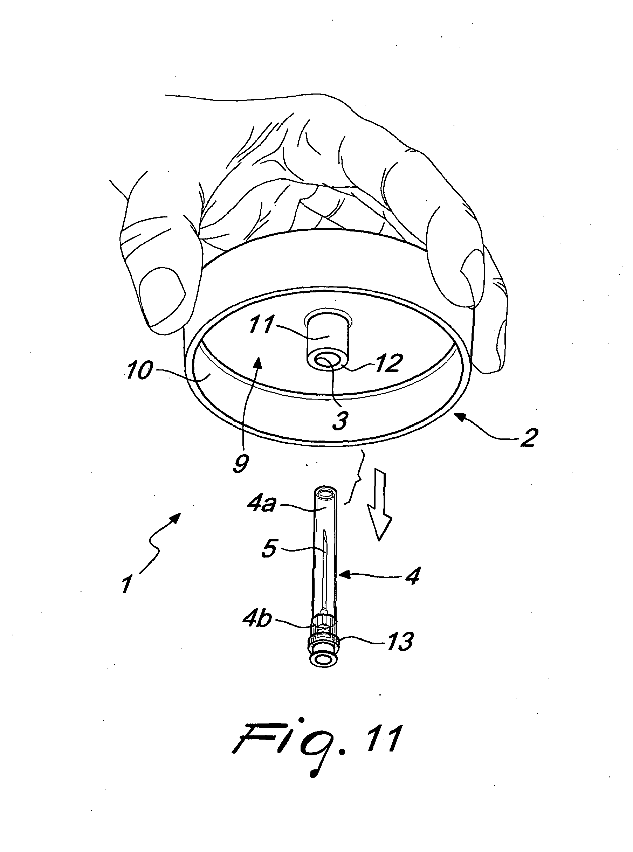

Moreover, as shown in Figures 9 to 11, beginning from both of the stable positions, it is always possible to decide later whether to dispose of the entire syringe 6 or whether to dispose of the recapped needle 5 separately from the rest of the syringe 6, by detaching the two parts with both hands again using the protection device 1.

In practice it has been found that the protection device with improved functionality, particularly for syringes and the like, according to the present invention, fully achieves the intended aim and object in that its geometry enables it to be arranged in two stable positions from which it is possible to perform the procedure of recapping the needle of the syringe using both hands or only one hand at the operator's discretion.

Indeed, the choice is linked to the operator's will and not to the geometric shape of the protection device, according to the invention, unlike the background art.

The choice of how to perform this operation is therefore left entirely to the operator who can change it at any time according to his/her needs.

It is not therefore a choice imposed by the device proper but one which depends only on the operator's will.

Whether using the device with one hand or with two hands the possibly contaminated surface is always the same and is never in any way in contact with either the operator or the surfaces upon which the device is rested.

By using the device according to the invention it is also possible to correctly perform a differentiated waste collection.

Such possibility relates to the use of the device as a recipient for containing other material (e.g. glass of a phial or used cotton) when it is placed on a surface for recapping with one hand only. Indeed in this case, once the needle is recapped it is possible, after lifting the device off the resting surface, to grip the cap of the syringe from below so as to prevent the contents of the device from falling and, with a small rotational movement, separate the recapped needle from the body and then dispose of the body in a first container and then, still holding the cap, it is possible, by overturning the device, to dispose of the contents in a second container and finally, by releasing the cap, to dispose of the recapped needle in a third container.

Another advantage of the protection device according to the present invention consists in that, when arranged with the rim turned upward, it is possible to use it as a recipient in which what is needed for performing the injection can be temporarily accommodated and therefore transported before performing it and, later, in which discarded material like vials, cotton or other materials can be disposed of temporarily after use, so as to not have to contaminate any other surface.

Another advantage of the protection device, according to the present invention, consists in that the flat face that comes into contact with the operator's hand or hands never comes into contact under any circumstances with parts of the syringe which are potentially contaminant, thus protecting the operator's hands from direct contact with parts of the syringe that are potentially contaminant.

Another advantage of the protection device, according to the present invention, consists in that the protruding position of the protection cap from the same side as the perimetric rim, as well as making it possible to recap the needle using two hands or only one hand, makes it more compact, and gives it a more homogeneous shape, thus making it better arrangeable and transportable in that it is free from surfaces that protrude outside the concave side of the protection device.

A further advantage of the protection device, according to the present invention, consists in that it is easier to handle and easier to clean thanks to its round shape, free from sharp corners and edges where dirt can become deposited.

Another advantage of the protection device, according to the present invention, consists in that it is economically competitive in comparison to the background art.

The protection device with improved functionality, particularly for syringes or the like, thus conceived, is susceptible of numerous modifications and variations, all of which are within the scope of the appended claims.

In addition, all the details may be replaced by other, technically equivalent elements.

In practice the materials employed, provided they are compatible with the specific use, and the contingent dimensions and shapes, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. A protection device (1) for the protection of an operator handling a syringe or the like that has a needle (5) and a needle covering cap (4), the device (1) comprising a supporting body (2) which is provided with a through hole (3) in which a needle covering cap (4) of a syringe (6) or the like is insertable, said supporting body (2) having a face (9) for the insertion of a said covering cap (4) in said hole (3) and at least one flat face (7) for the resting contact of said supporting body (2) on a resting surface (8) or the like, the flat face (7) being arranged on the opposite side of said supporting body (2) with respect to said face (9), said face (9) that lies opposite said flat face (7) being provided with a raised perimetric containment rim (10), said hole (3) having a diameter that is substantially larger than the diameter of the central portion (4a) of a said covering cap (4) and substantially smaller than the diameter of the end portion (4b) of said covering cap (4) located at its end for the insertion therein of said needle (5), a collar (11) being provided around said hole (3), said collar extending from said face (9) for the insertion of the covering cap (4), **characterized in that** it is shaped so as to allow grasping with one hand whose fingers grip onto said perimetric containment rim (10), said collar (11) extending from said at least one flat face (7) for a length that is shorter than the height of said raised perimetric containment rim (10) so as to allow resting contact of said supporting body (2) in a stable position, with said at least one flat face (7) directed upward, on said supporting surface (8) or the like.

2. The protection device according to claim 1, **characterized in that** said supporting body (2) is substantially disc-like.

3. The protection device according to one or more of the preceding claims, **characterized in that** said collar (11) is arranged substantially at right angles to said at least one flat face (7) and to said face (9) that lies opposite said flat face.

4. The protection device according to one or more of the preceding claims, **characterized in that** said diameter of said hole (3) is such as to allow an engagement by mechanical interference with the needle insertion end portion (4b) of a covering cap (4) that is radially larger than the central portion (4a) thereof, said covering cap (4) having only a conicalness concordant with a direction of insertion thereof in said hole (3).

5. The protection device according to one or more of the preceding claims, **characterized in that** said hole (3) is coaxial to said collar (11).

6. The protection device according to one or more of the preceding claims, **characterized in that** said hole (3) is arranged at the center of said supporting body (2).

7. A method for removing the cap from a needle of a syringe by means of a device according to one or more of the preceding claims, **characterized in that** it comprises the steps of:
- grasping the device (1) with one hand whose fingers grip onto its perimetric containment rim (10);
- holding a syringe (6) having a needle (5) and a needle covering cap (4) with the other hand and inserting the needle (5) with the associated covering cap (4) into the hole (3), guided by the collar (11) provided around said hole (3) and extending from the face (9) for the insertion of the covering cap (4), and making the covering cap (4) protrude from the flat surface (7) of the device that is opposite to the the face (9) for the insertion;
- gripping the covering cap (4) with the fingers, at the central portion (4a) opposite to the needle insertion end portion (4b), and removing, with the hand holding the syringe (6), the needle from the covering cap (4).

8. A method for recapping a needle of a syringe with the aid of both hands by means of a device according to one or more of claims 1 to 6, **characterized in that** it comprises the steps of:
- placing the device (1), with a needle covering cap (4) of a syringe (6) inserted in the hole (3), in a stable position on a resting surface, with the device turned with its flat face (7) facing upward and with the covering cap (4) protruding upward from the hole (3);
- gripping the device (1) with one hand by means of the covering cap (4), and capping the needle (5) of the syringe (6) with the other hand;
- separating the syringe (6) from the needle (5) inserted in the covering cap (4);
- disposing of the capped needle in a container for sharps waste.

9. A method for recapping a needle of a syringe with the aid of one hand only by means of a device according to one or more of claims 1 to 6, **characterized in that** it comprises the steps of:
- placing the device (1), with the needle covering cap (4) of a syringe (6) inserted in the hole (3), in a stable position on a resting surface, with the device turned with its flat face (7) facing the resting surface and with the covering cap (4) protruding upward from the hole (3);
- gripping the syringe (6) with one hand and inserting its needle (5) into the covering cap (4);
- lifting the device (1) with one hand from the resting surface so as to make the covering cap (4) protrude from the flat face (7);
- gripping the covering cap (4) with one hand and separating the syringe (6) from the needle (5) inserted in the covering cap (4) with the other hand;
- disposing of the capped needle in a container for sharps waste.

## Patentansprüche

1. Eine Schutzvorrichtung (1) zum Schutz eines Bedieners, der eine Spritze oder Ähnliches handhabt, die eine Nadel (5) und eine Nadel-Abdeckkappe (4) hat, wobei die Vorrichtung (1) einen tragenden Körper (2) hat, der mit einer Durchgangsbohrung (3) versehen ist, in welche eine Nadel-Abdeckkappe (4) einer Spritze (6) oder dergleichen einsetzbar ist, wobei der tragende Körper (2) eine Flache (9) zum Einsetzen der Abdeckkappe (4) in die Bohrung (3) und mindestens eine flache Flache (7) für den Auflagekontakt des tragenden Körpers (2) auf einer Auflageflache (8) oder dergleichen hat, wobei die flache Flache (7) auf der Seite des tragenden Körpers (2) angeordnet ist, die der Flache (9) gegenüberliegt, und die Flache (9), die der flachen Flache (7) gegenüberliegt, mit einem erhöhten perimetrischen Einfassungsrand (10) versehen ist, wobei die Bohrung (3) einen Durchmesser hat, der wesentlich größer ist als der Durchmesser des zentralen Abschnitts (4a) der Abdeckkappe (4) und wesentlich kleiner als der Durchmesser des Endabschnitts (4b) der Abdeckkappe (4), der sich an ihrem Ende zum Einführen der Nadel (5) in denselben befindet, wobei ein Bund (11) um die Bohrung (3) herum angebracht ist, der sich von der Flache (9) zum Einsetzen der Abdeckkappe (4) erstreckt, **dadurch gekennzeichnet, dass** er geformt ist, um das Greifen mit einer Hand zu ermöglichen, deren Finger den perimetrischen Einfassungsrand (10) greifen, wobei der Bund (11) sich von der mindestens einen flachen Flache (7) über eine Länge erstreckt, die kürzer ist als die Höhe des erhöhten perimetrischen Einfassungsrandes (10), um so den Auflagekontakt des tragenden Körpers (2) in einer stabilen Position, mit der mindestens einen flachen Flache (7) nach oben gerichtet, auf der Auflageflache (8) oder dergleichen zu ermöglichen.

2. Die Schutzvorrichtung gemäß Anspruch 1, **dadurch gekennzeichnet, dass** der tragende Körper (2) im Wesentlichen scheibenförmig ist.

3. Die Schutzvorrichtung gemäß einem oder mehreren der obigen Ansprüche, **dadurch gekennzeichnet, dass** der Bund (11) im Wesentlichen in rechten Winkeln zu der mindestens einen flachen Fläche (7) und zu der Fläche (9) angeordnet ist, die gegenüber der flachen Fläche liegt.

4. Die Schutzvorrichtung gemäß einem oder mehreren der obigen Ansprüche, **dadurch gekennzeichnet, dass** der Durchmesser der Bohrung (3) derart ist, dass er einen mechanischen Eingriff mit dem Nadeleinführ-Endabschnitt (4b) einer Abdeckkappe (4) ermöglicht, der radial größer ist als der zentrale Abschnitt (4a) davon, wobei die Abdeckkappe (4) nur eine Kegelform hat, die mit einer Einführrichtung derselben in die Bohrung (3) übereinstimmt.

5. Die Schutzvorrichtung gemäß einem oder mehreren der obigen Ansprüche, **dadurch gekennzeichnet, dass** die Bohrung (3) koaxial mit dem Bund (11) ist.

6. Die Schutzvorrichtung gemäß einem oder mehreren der obigen Ansprüche, **dadurch gekennzeichnet, dass** die Bohrung (3) in der Mitte des tragenden Körpers (2) angebracht ist.

7. Ein Verfahren zum Entfernen der Kappe von einer Nadel einer Spritze mit Hilfe einer Vorrichtung gemäß einem oder mehreren der obigen Ansprüche, **dadurch gekennzeichnet, dass** es folgende Schritte umfasst:
- Greifen der Vorrichtung (1) mit einer Hand, deren Finger ihren perimetrischen Einfassungsrand (10) greifen,
- Halten einer Spritze (6), die eine Nadel (5) und eine Nadel-Abdeckkappe (4) hat, mit der anderen Hand und Einführen der Nadel (5) mit der dazugehörigen Abdeckkappe (4) in die Bohrung (3), geführt von dem Bund (11), der um die Bohrung (3) herum angebracht ist und sich von der Fläche (9) zum Einführen der Abdeckkappe (4) erstreckt, und Veranlassen, dass die Abdeckkappe (4) aus der flachen Oberfläche (7) der Vorrichtung herausragt, die der Flache (9) zum Einführen gegenüberliegt,
- Greifen der Abdeckkappe (4) mit den Fingern, im zentralen Abschnitt (4a), der dem Nadeleinfuhr-Endabschnitt (4b) gegenüberliegt, und Entfernen der Nadel von der Abdeckkappe (4) mit der Hand, die die Spritze (6) halt.

8. Ein Verfahren zum Wiederaufsetzen der Kappe auf eine Nadel einer Spritze mit Hilfe beider Hände durch eine Vorrichtung gemäß einem oder mehreren der Ansprüche 1 bis 6, **dadurch gekennzeichnet, dass** es folgende Schritte umfasst:
- Platzieren der Vorrichtung (1), mit einer Nadel-Abdeckkappe (4) einer Spritze (6), die in die Bohrung (3)eingeführt ist, in eine stabile Position auf einer Auflagefläche, wobei die Vorrichtung mit ihrer flachen Flache (7) nach oben weisend gedreht ist und wobei die Abdeckkappe (4) aus der Bohrung (3) nach oben ragt,
- Greifen der Vorrichtung (1) mit einer Hand mit Hilfe der Abdeckkappe (4) und Aufsetzen der Kappe auf die Nadel (5) der Spritze (6) mit der anderen Hand,
- Trennen der Spritze (6) von der Nadel (5), die in die Abdeckkappe (4) eingefuhrt ist,
- Entsorgen der mit der Kappe versehenen Nadel in einem Behälter fur spitze Abfalle.

9. Ein Verfahren zum Wiederaufsetzen der Kappe auf eine Nadel einer Spritze mit Hilfe nur einer Hand durch eine Vorrichtung gemäß einem oder mehreren der Ansprüche 1 bis 6, **dadurch gekennzeichnet, dass** es folgende Schritte umfasst:
- Platzieren der Vorrichtung (1), mit der Nadel-Abdeckkappe (4) einer Spritze (6), die in die Bohrung (3) eingeführt ist, in eine stabile Position auf einer Auflagefläche, wobei die Vorrichtung so gedreht ist, dass ihre flache Flache (7) der Auflageflache zugewandt ist, und wobei die Abdeckkappe (4) aus der Bohrung (3) nach oben heraus ragt,
- Greifen der Spritze (6) mit einer Hand und Einführen ihrer Nadel (5) in die Abdeckkappe (4),
- Anheben der Vorrichtung (1) von der Auflageflache mit einer Hand, um die Abdeckkappe (4) aus der flachen Flache (7) herausragen zu lassen,
- Greifen der Abdeckkappe (4) mit einer Hand und Trennen der Spritze (6) von der in die Abdeckkappe (4) eingesetzten Nadel (5) mit der anderen Hand,
- Entsorgen der mit der Kappe versehenen Nadel in einem Behalter für spitze Abfalle.

## Revendications

1. Dispositif de protection (1) pour protéger un opérateur qui manipule une seringue ou similaire qui possède une aiguille (5) et un capuchon de recouvrement d'aiguille (4), le dispositif (1) comprenant un corps de support (2) qui est prévu avec un trou débouchant (3) dans lequel un capuchon de recouvrement d'aiguille (4) d'une seringue (6) ou similaire peut être inséré, ledit corps de support (2) ayant une face (9) pour l'insertion dudit capuchon de recouvrement (4) dans ledit trou (3) et au moins une face plate (7) pour le contact au repos dudit corps de support (2) sur une surface de repos (8) ou similaire, la face plate (7) étant agencée sur le côté opposé dudit corps de support (2) par rapport à ladite face (9), ladite face (9) qui est à l'opposé de ladite face plate (7) étant prévue avec un rebord de confinement périmétral relevé (10), ledit trou (3) ayant un diamètre qui est sensiblement plus grand que le diamètre de la partie centrale (4a) dudit capuchon de recouvrement (4) et sensiblement inférieur au diamètre de la partie d'extrémité (4b) dudit capuchon de recouvrement (4) positionné au niveau de son extrémité pour l'insertion dans ce dernier de ladite aiguille (5), un collier (11) étant prévu autour dudit trou (3), ledit collier s'étendant à partir de ladite face (9) pour l'insertion du capuchon de recouvrement (4), **caractérisé en ce qu'**il est formé afin de permettre la saisie avec une main dont les doigts saisissent ledit rebord de confinement périmétral (10), ledit collier (11) s'étendant à partir de ladite au moins une face plate (7) sur une longueur qui est plus courte que la hauteur dudit rebord de confinement périmétral relevé (10) afin de permettre le contact au repos dudit corps de support (2) dans une position stable, avec ladite au moins une face plate (7) dirigée vers le haut, sur ladite surface de support (8) ou similaire.

2. Dispositif de protection selon la revendication 1, **caractérisé en ce que** ledit corps de support (2) est sensiblement en forme de disque.

3. Dispositif de protection selon une ou plusieurs des revendications précédentes, **caractérisé en ce que** ledit collier (11) est agencé sensiblement en angle droit par rapport à ladite au moins une face plate (7) et à ladite face (9) qui est à l'opposé de ladite face plate.

4. Dispositif de protection selon une ou plusieurs des revendications précédentes, **caractérisé en ce que** ledit diamètre dudit trou (3) est tel qu'il permet une mise en prise par interférence mécanique avec la partie d'extrémité d'insertion d'aiguille (4b) d'un capuchon de recouvrement (4) qui est radialement plus grande que sa partie centrale (4a), ledit capuchon de recouvrement (4) ayant uniquement une absence de conicité concordant avec sa direction d'insertion dans ledit trou (3).

5. Dispositif de protection selon une ou plusieurs des revendications précédentes, **caractérisé en ce que** ledit trou (3) est coaxial par rapport audit collier (11).

6. Dispositif de protection selon une ou plusieurs des revendications précédentes, **caractérisé en ce que** ledit trou (3) est agencé au centre dudit corps de support (2).

7. Procédé pour retirer le capuchon d'une aiguille d'une seringue au moyen d'un dispositif selon une ou plusieurs des revendications précédentes, **caractérisé en ce qu'**il comprend les étapes consistant à :
saisir le dispositif (1) avec une main dont les doigts saisissent son rebord de confinement périmétral (10) ;
maintenir une seringue (6) ayant une aiguille (5) et un capuchon de recouvrement d'aiguille (4) avec l'autre main et insérer l'aiguille (5) avec le capuchon de recouvrement (4) associé dans le trou (3), guidé par le collier (11) prévu autour dudit trou (3) et s'étendant à partir de la face (9) pour l'insertion du capuchon de recouvrement (4), et faire faire saillie au capuchon de recouvrement (4) de la surface plate (7) du dispositif qui est opposée à la face (9) pour l'insertion ;
saisir le capuchon de recouvrement (4) avec les doigts, au niveau de la partie centrale (4a) opposée à la partie d'extrémité d'insertion d'aiguille (4b), et retirer avec la main qui maintient la seringue (6), l'aiguille du capuchon de recouvrement (4).

8. Procédé pour recouvrir avec un capuchon une aiguille d'une seringue à l'aide des deux mains au moyen d'un dispositif selon une ou plusieurs des revendications 1 à 6, **caractérisé en ce qu'**il comprend les étapes consistant à :
placer le dispositif (1), avec un capuchon de recouvrement d'aiguille (4) d'une seringue (6) inséré dans le trou (3), dans une position stable sur une surface de repos, avec le dispositif orienté avec sa face plate (7) vers le haut et avec le capuchon de recouvrement (4) faisant saillie vers le haut par rapport au trou (3) ;
saisir le dispositif (1) avec une main, au moyen du capuchon de recouvrement (4), et mettre le capuchon sur l'aiguille (5) de la seringue (6) avec l'autre main ;
séparer la seringue (6) de l'aiguille (5) insérée dans le capuchon de recouvrement (4) ;
jeter l'aiguille recouverte avec un capuchon dans un récipient pour les déchets tranchants.

9. Procédé pour recouvrir avec un capuchon une aiguille d'une seringue à l'aide d'une main uniquement au moyen d'un dispositif selon une ou plusieurs des revendications 1 à 6, **caractérisé en ce qu'**il comprend les étapes consistant à :
placer le dispositif (1), avec le capuchon de recouvrement d'aiguille (4) d'une seringue (6) inséré dans le trou (3), dans une position stable sur une surface de repos, avec le dispositif orienté avec sa face plate (7) faisant face à la surface de repos et avec le capuchon de recouvrement (4) faisant saillie vers le haut par rapport au trou (3) ;
saisir la seringue (6) d'une main et insérer son aiguille (5) dans le capuchon de recouvrement (4) ;
lever le dispositif (1) avec une main par rapport à la surface de repos afin de faire faire saillie au capuchon de recouvrement (4) par rapport à la face plate (7) ;
saisir le capuchon de recouvrement (4) avec une main et séparer la seringue (6) de l'aiguille (5) insérée dans le capuchon de recouvrement (4) avec l'autre main ;
jeter l'aiguille recouverte avec un capuchon dans un récipient pour les déchets tranchants.
